# EUROPEAN PATENT SPECIFICATION

(11) **EP 1 902 139 B1**
(45) Date of publication and mention of the grant of the patent: **26.01.2011**
(21) Application number: 06787380.2
(22) Date of filing: 12.07.2006
(51) Int. Cl.: C12P 7/02, C12P 7/40

(54) **METHOD FOR CONVERTING BIOMASS TO CARBOXYLIC ACIDS**
VERFAHREN ZUR UMWANDLUNG VON BIOMASSE IN CARBONSÄUREN
PROCÉDÉ DE CONVERSION DE BIOMASSE EN ACIDES CARBOXYLIQUES

(30) Priority: 12.07.2005 US 698751 P; 11.07.2006 US 456653
(43) Date of publication of application: 26.03.2008
(73) Proprietor: The Texas A&M University System, College Station, TX 77843-3369 (US)
(72) Inventor: HOLTZAPPLE, Mark, T., College Station, TX 77840 (US); DAVISON, Richard, R., Bryan, TX 77801 (US); GRANDA, Cesar, B., College Station, TX 77845 (US); AGBOGBO, Frank, K., Wake Forest, NC 27587 (US); FU, Zhihong (nmi), College Station, TX 77840-1674 (US)
(74) Representative: Dauncey, Mark Peter
(86) International application number: PCT/US2006/027466
(87) International publication number: WO 2007/009085

(56) References cited:
- WO-A-2005/044743
- AIELLO-MAZZERI ET AL: "Conversion of municipal solid waste to carboxylic acids using a mixed culture of mesophilic microorganisms" BIORESOURCE TECHNOLOGY, vol. 97, 12 April 2005 (2005-04-12), pages 47-56, XP002410338
- AIELLO-MAZZARRI ET AL: "Conversion of municipal solid waste to carboxylic acids by anaerobic countercurrent fermentation: Effect of using intermediate lime treatment" POSTER PRESENTATION 3B-46, 27TH SYMPOSIUM ON BIOTECHNOLOGY FOR FUELS AND CHEMICALS, May 2005 (2005-05), page 1, XP002410274 Retrieved from the Internet: URL:http://www1.eere.energy.gov/biomass/bi otech_symposium/session3b_pp.html> [retrieved on 2006-12-04]
- N.N.: "The MixAlco Process" ALLEXPERTS, pages 1-2, XP002410275 Retrieved from the Internet: URL:http://experts.about.com/e/m/mi/mixalc o_process.htm> [retrieved on 2006-11-23]
- HOLTZAPPLE, [Online] 2006, Retrieved from the Internet: <URL:http://engineering.tamu.edu/research/l ectures/>

## Description

### TECHNICAL FIELD

The present invention relates generally to biomass processing and, more specifically, to methods for converting biomass into carboxylic acids and alcohols.

### BACKGROUND

A great deal of biomass, particularly lignocellulosic biomass, remains unused or inefficiently used during agricultural and industrial processes. Disposal of this biomass is often difficult or costly. Therefore, methods of using this biomass to produce useful chemicals are quite valuable. Organic acids are one example of such useful chemicals. Historically, organic acids were produced from animal fat or vegetable oil sources or from petroleum sources in substantially nonaqueous systems. More recently, organic acids have been identified as among the most attractive products for manufacture from biomass by fermentation. Alcohols are also important industrial chemicals that may be produced by fermentation of biomass. However, extraction of organic acids and alcohols from the overall fermentation product is not easy and is often inefficient in the use of energy, water, and reactant chemicals. Examples of previous techniques can be found in WO 2005/044743 where carbon dioxide or a carbon dioxide-containing gas mixture and ammonia or ammonia-containing gas mixture are combined to form buffer compounds; and in "Conversion of Municipal Solid Waste to Carboxylic Acids using a Mixed Culture of Mesophilic Microorganisms" published by Aiello-Mazzarri, et al., BioResource technology, Vol. 97, 12 April 2005, pages 47-56.

### SUMMARY

In accordance with the teachings of the present invention, a method for converting biomass into useful chemicals are provided. In a particular embodiment, the method comprises fermenting biomass in one or more fermentors to produce a fermentation broth comprising ammonium carboxylate salt, the fermentors containing a buffer selected from the group consisting of ammonium carbonate and ammonium bicarbonate. The method further comprises reacting the ammonium carboxylate salt with a high-molecular-weight amine to produce amine carboxylate salt, and thermally cracking the amine carboxylate salt to produce carboxylic acid. In another embodiment, the method comprises reacting the ammonium carboxylate salt from the fermentors with a low-molecular-weight amine to produce a low-molecular-weight-amine carboxylate salt, switching the low-molecular-weight amine in the low-molecular-weight-amine carboxylate salt with a high-molecular-weight amine to form a high-molecular-weight-amine carboxylate salt, and thermally cracking the high-molecular-weight-amine carboxylate salt to produce carboxylic acid. In yet another embodiment, the method comprises reacting the ammonium carboxylate salt from the fermentors with a high-molecular-weight alcohol to produce a high-molecular-weight ester, and hydrogenating the high-molecular weight ester to produce alcohol.

A technical advantage of particular embodiments may include the ability to buffer the fermentation reaction using ammonium carbonate or ammonium bicarbonate. If ammonia were added directly to the reactions, the pH may become too high and damage the microorganisms used to ferment the biomass. The use of ammonium carbonate or ammonium bicarbonate lessens or eliminates this problem. Additionally, the use of ammonium carbonate or ammonium bicarbonate buffers allows for simplified downstream processing of the fermentation broth, compared to calcium-based buffer systems. Such calcium-based buffer system may result in the formation of calcium salts that collect on the surfaces of heat exchangers and other equipment. In contrast, the ammonium salts of the present invention do not tend to collect on equipment surfaces.

Another technical advantage of particular embodiments may include the ability to reduce or eliminate solids handling during downstream processing.

### BRIEF DESCRIPTION OF THE DRAWINGS

For a more complete understanding of the present invention and features and advantages thereof, reference is now made to the following description, taken in conjunction with the accompanying drawings, in which:
FIGURE 1 illustrates a system for converting biomass into carboxylic acid according to a particular method of the present invention;
FIGURE 2 illustrates a flowchart of a method of converting biomass into carboxylic acid using the system shown in FIGURE 1;
FIGURE 3 illustrates a system for converting biomass into carboxylic acid according to a particular method of the present invention;
FIGURE 4 illustrates a flowchart of a method of converting biomass into carboxylic acid using the system shown in FIGURE 3;
FIGURE 5 illustrates a system for converting biomass to alcohol according to a particular method of the present invention; and
FIGURE 6 illustrates a flowchart of a method of converting biomass into alcohol using the system shown in FIGURE 5.

### DETAILED DESCRIPTION OF EXAMPLE EMBODIMENTS

In accordance with the teachings of the present invention, a method for converting biomass into useful chemicals are provided. In a particular embodiment, the method comprises fermenting biomass in one or more fermentors to produce a fermentation broth comprising ammonium carboxylate salts, the fermentors containing an ammonium carbonate or ammonium bicarbonate buffer. The method further comprises reacting the ammonium carboxylate salts from the fermentors with a high-molecular-weight amine to produce amine carboxylate salt, and thermally cracking the amine carboxylate salt to produce carboxylic acid. In another embodiment, the ammonium carboxylate salts from the fermentors may be reacted with a low-molecular-weight amine to produce a low-molecular-weight-amine carboxylate salt. The low-molecular-weight amine in the low-molecular-weight-amine carboxylate salt may then be switched with a high-molecular-weight amine to form a high-molecular-weight-amine carboxylate salt, which is then thermally cracked to produce carboxylic acid. In yet another embodiment, the ammonium carboxylate salts from the fermentors may be reacted with a high-molecular-weight alcohol to produce a high-molecular-weight ester, which may be hydrogenated to produce alcohol. In particular embodiments, the use of ammonium carbonate or ammonium bicarbonate as a buffer in the fermentors allows for alternative downstream processing methods for producing carboxylic acids, esters, and alcohols. Moreover, particular embodiments may allow for simplified recovery of carboxylic acids and/or alcohols from the fermentation broth.

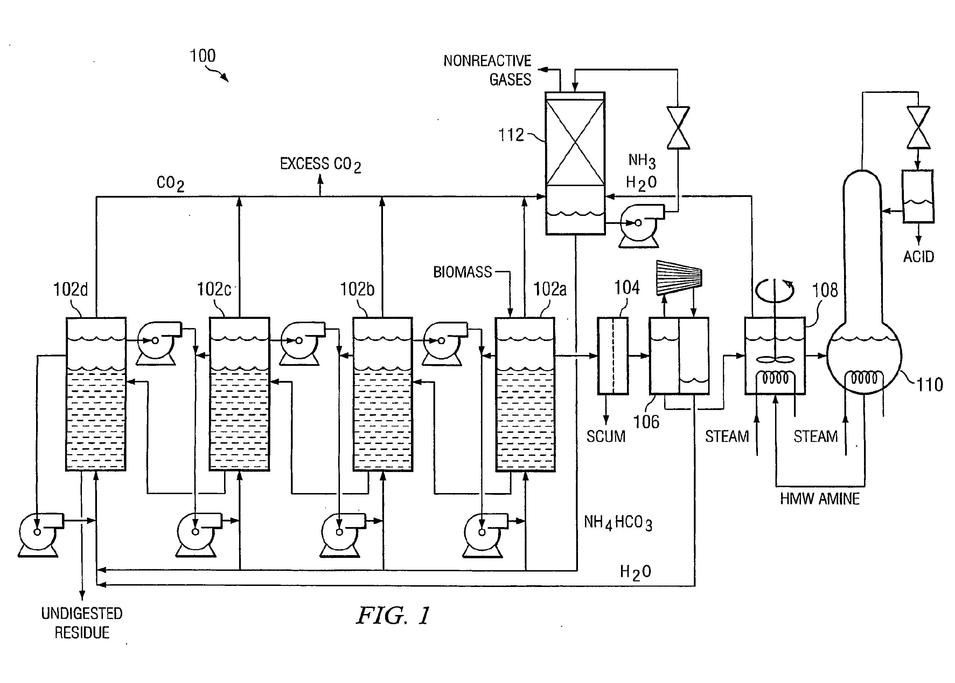

FIGURE 1 illustrates a fermentation system 100 in accordance with a particular method of the present invention. Fermentation system 100 is a fermentation system that may be used to produce carboxylic acids from biomass. Generally, fermentation system 100 comprises one or more fermentors 102, a dewatering system 106, a reactor 108, distillation column 110, and a packed column 112. As shown in FIGURE 1, fermentation system 100 comprises four countercurrent fermentors 102a-d, although any number of suitable fermentor geometries and arrangements may be used. These four fermentors 102a-d comprise a countercurrent fermentor system in which fresh biomass is added to the top of fermentor 102a and fresh water is added to the bottom of fermentor 102d, and the biomass and water move through the fermentors 102 in opposite directions. For example, undigested residues removed from the bottom of fermentor 102a are sent to fermentor 102b, undigested residues removed from the bottom of fermentor 102b are sent to fermentor 102c, undigested residues from the bottom of fermentor 102c are sent to fermentor 102d, and undigested residues from the bottom of fermentor 102d are removed from the fermentation system and discarded. Meanwhile, liquid from fermentor 102d is sent to fermentor 102c, liquid from fermentor 102c is sent to fermentor 102b, liquid from 102b is sent to fermentor 102a, and fermentation broth is ultimately harvested from fermentor 102a.

In particular embodiments, a screw press (not illustrated) or other suitable dewatering device may be used to reduce the liquid content in the solids that are transferred between the various fermentors 102. Furthermore, each fermentor 102 may be equipped with a circulation loop to facilitate the distribution of a methane inhibitor, such as iodoform, bromoform, and bromoethane sulfonic acid, and/or a buffer, such as ammonium bicarbonate or ammonium carbonate, through the solid mass. In particular embodiments, the addition of the methane inhibitor may be optional, as the ammonium ion is already a very effective inhibitor of methanogens.

Inside fermentors 102, a mixed culture of acid-forming microorganisms facilitate the fermentation of the biomass. Although a variety of suitable microorganisms may be used, particular embodiments utilize microorganisms adapted to high-salt environments, such as inoculum from marine environments or salt lakes. Other embodiments may utilize microorganisms native to soil or cattle rumen. These microorganisms may survive over a fairly broad pH range (*e.g*., 5.0 to 8.0); however, in particular embodiments the fermentation is most effective when the pH is near neutrality (*i.e*., 6.5 to 7.5). Accordingly, the temperature and pH inside fermentors 102 may be controlled in any suitable manner. For example, in particular embodiments the temperatures inside fermentors 102 may be controlled by regulating the temperature of the circulating liquid. The pH insides fermentors 102 may be regulated by the addition rate of buffer. In particular embodiments, this buffer may comprise ammonium carbonate or ammonia bicarbonate.

Fermentation broth harvested from fermentor 102 is further processed downstream. In particular embodiments, this fermentation broth may include scum that is undesirable in the downstream processing steps. Therefore, particular embodiments may employ a variety of methods to remove this scum. For example, in particular embodiments, the fermentation broth may be pumped through an ultrafilter 104 having a molecular weight cut-off that allows ammonium carboxylic acid salts to pass but that retains the scum. In other embodiments, a coagulant or flocculant, such as those employed to clarify sugar juice extracted from sugarcane, may be added the fermentation broth to cause a precipitate to form that may be removed by filtration.

Regardless of the method (if any) of de-scumming the fermentation broth, the fermentation broth from fermentors 102 is passed to a dewatering system 106, which removes water from the broth to form a nearly saturated (*i.e*., approximately 50%) solution of ammonium carboxylate salts. Although a variety of dewatering systems may be used in accordance with the teachings of the present invention, FIGURE 1 illustrates dewatering system 106 as a vapor-compression system. In this system 106, vapors from the concentrated salt solution are compressed, allowing them to condense in a heat exchanger. The heat of condensation in the condenser, in turn, provides the heat of evaporation in the boiler. In this manner, heat is recycled in the system. Only a small amount of shaft work provided to the compressor is needed to drive the system.

The concentrated ammonium carboxylate salts from dewatering system 106 are sent to a heated, well-mixed reactor 108 where a high-molecular-weight ("HMW") amine is added to the solution to react to form HMW-amine carboxylate salts. In particular embodiments, the HMW amine added comprises tri-octyl amine. In other embodiments, triethanol amine may be reacted with a HMW carboxylic acid to make the corresponding ester. In particular embodiments a surfactant may also be added to facilitate contact between the amine phase and the water phase.

Heating reactor 108 drives off both water and ammonia from the solution, the water and ammonia having been displaced by the HMW amine to form HMW-amine carboxylate salt. This ammonia and water from reactor 108 is sent to a packed column 112 where it reacts with carbon dioxide from fermentors 102 to form ammonium bicarbonate or ammonium carbonate, depending upon the pH maintained with the column. The ammonium bicarbonate or ammonium carbonate may then be used as the buffer in fermentors 102. In particular embodiments, this ammonium bicarbonate or ammonium carbonate may be concentrated before it is sent to fermentors 102 to help reduce the water load sent to the fermentors.

The HMW-amine carboxylate salts from reactor 108 are sent to a reactive distillation column 110 where they are thermally cracked to produce carboxylic acids, which exit from the top of column 110, and HMW amines, which exit from the bottom of column 110 and are recycled into reactor 108. At 1 atm, typical cracking temperatures are from about 150°C to about 200°C, depending on the molecular weight of the carboxylic acid. The higher the molecular weight of the acid, the higher the temperature required for thermal cracking to occur. The carboxylic acids exiting column 110 may then be collected.

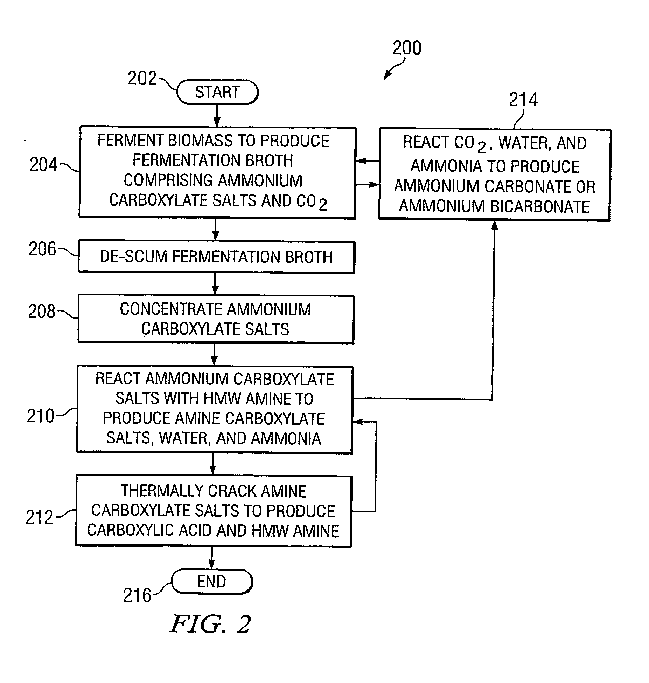

A better understanding of the process employed by fermentation system 100 may be had by making reference to FIGURE 2, which illustrates a flowchart 200 of a method of producing carboxylic acids from biomass utilizing the same equipment as shown in FIGURE 1. Flowchart 200 begins at step 202. At step 204, biomass is fermented to produce carbon dioxide and a fermentation broth comprising ammonium carboxylate salts. Generally, this is performed using a plurality of countercurrent fermentors utilizing an ammonium carbonate or ammonia bicarbonate buffer. The fermentation broth produced by the plurality of fermentors is then de-scummed at step 206. In particular embodiments, this may be performed using an ultrafilter that filters out the scum or a coagulant or flocculant that causes the scum to form a precipitate that may then be filtered out.

At step 208, the de-scummed fermentation broth is then concentrated using a dewatering system, such as a vapor-compression system. This dewatering system concentrates the fermentation broth into a nearly saturated (*i.e*., approximately 50%) solution of ammonium carboxylate salts. This nearly saturated solution of ammonium carboxylate salts is then reacted with a HMW amine in a heated, well-mixed reactor to produce amine carboxylate salts at step 210. As part of this process, water and ammonia are also produced. At step 214, this water and ammonia is reacted with carbon dioxide given off by the plurality of fermentors to produce ammonium carbonate or ammonium bicarbonate that may be used to buffer the fermentation reaction inside the plurality of countercurrent fermentors.

The amine carboxylate salts produced at step 210 are then thermally cracked in a reactive distillation column to produce carboxylic acid and HMW amine at step 212. HMW amine exits the bottom of the column and may be used to react with the ammonium carboxylate salts at step 210. The carboxylic acid, on the other hand, exits the top of the column and may be collected. At step 216, the flowchart 200 terminates.

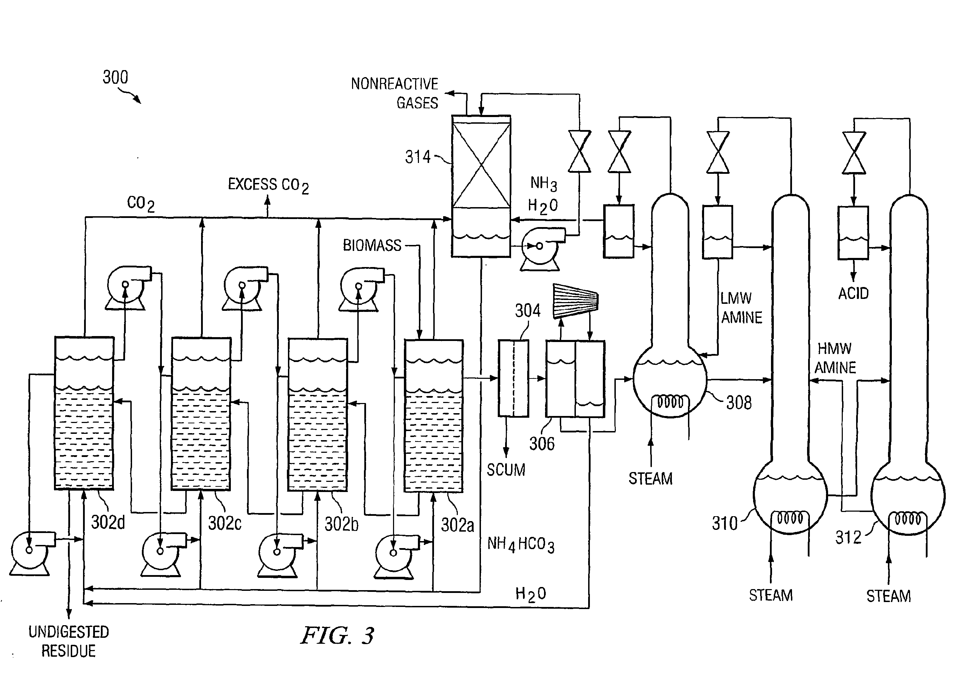

FIGURE 3 illustrates a fermentation system 300 in accordance with another embodiment. Similar to fermentation system 100 (FIGURE 1), fermentation system 300 may be used to produce carboxylic acids from biomass. However, unlike fermentation system 100, which only utilizes HMW amine, fermentation system 300 also utilizes a low-molecular-weight ("LMW") amine, such as triethyl amine, methyl diethyl amine, dimethyl ethanol amine, or ethanol amine, to produce carboxylic acids.

Generally, fermentation system 300 comprises one or more fermentors 302, a dewatering system 306, distillation columns 308, 310, and 312, and a packed column 314. Although any number of suitable fermentor geometries and arrangements may be used in accordance with the teachings of the present invention, FIGURE 3 illustrates fermentation system 300 comprising four countercurrent fermentors 302a-d in which fresh biomass is added to the top of fermentor 302a and fresh water is added to the bottom of fermentor 302d. These fermentors 302 operable similarly to fermentors 102 described above with regard to FIGURE 1.

Fermentation broth harvested from fermentor 302a is sent for downstream processing. In particular embodiments, this fermentation broth may also include scum that is undesirable in the downstream processing steps. In particular embodiments, this scum may be removed using any suitable method. For example, in particular embodiments, the fermentation broth may be pumped through an ultrafilter 304 having a molecular weight cut-off that allows ammonium carboxylic acid salts to pass but retains scum. In other embodiments, a coagulant or flocculant, such as those employed to clarify sugar juice extracted from sugarcane, may be added to the fermentation broth to cause a precipitate to form that is removable by suitable filtration.

Regardless of the method (if any) of de-scumming, the fermentation broth from fermentors 302 is passed to a dewatering system 306, which removes water from the broth to form a nearly saturated solution (*i.e*., about 50%) of ammonium carboxylate salts. Although a variety of dewatering systems may be used, FIGURE 3 illustrates dewatering system 306 as a vapor-compression system. This vapor-compression system works similarly to dewatering system 106 discussed above with regard to FIGURE 1.

The concentrated ammonium carboxylate salts from dewatering system 306 are sent to distillation column 308, where a LMW amine is added to produce LMW-amine carboxylate salts, driving off water and ammonia in the process. In particular embodiments, the LMW amine added may comprise triethyl amine, methyl diethyl amine, dimethyl ethanol amine, ethanol amine, or any other suitable LMW amine. In particular embodiments, the LMW amine is a water-soluble amine having a standard boiling point above about 100°C so that the amine is less volatile than water. Moreover, in particular embodiments, the LMW may be a tertiary amine, helping to avoid possible amide formation. Regardless of the selected LMW amine, the top of column 308 has a partial condenser that sends reflux (primarily water) back into the column to prevent the loss of LMW amine vapors. The ammonia and water not sent back to column 308 are sent to a packed column 314 where they react with carbon dioxide from fermentors 302 to form ammonium bicarbonate or ammonium carbonate, depending upon the pH maintained with the column, which may be used as a buffer in fermentors 302. In particular embodiments, this ammonium bicarbonate or ammonium carbonate may be concentrated before it is sent to fermentors 302 to help reduce the water load sent to the fermentors.

The bottoms of distillation column 308 are sent to distillation column 310, where the LMW amine in the LMW-amine carboxylate salt is switched with a HMW amine to produce HMW-amine carboxylate salts and LMW amine. The LMW amine exits the top of column 310 and is recycled to column 308. In particular embodiments, to avoid thermal cracking or amide formation, column 308 may be operated under vacuum to reduce the temperature inside the column. HMW-amine carboxylate salts exit the bottom of the second column and enter reactive distillation column 312.

Inside reactive distillation column 312, the HMW-amine carboxylate salts are thermally cracked to produce carboxylic acids, which exit from the top of the column, and HMW amine, which exits from the bottom of the column and is recycled into distillation column 310. At 1 atm, typical cracking temperatures are from about 150°C to about 200°C, depending on the molecular weight of the carboxylic acid. The higher the molecular weight of the acid, the higher the temperature required for thermal cracking to occur.

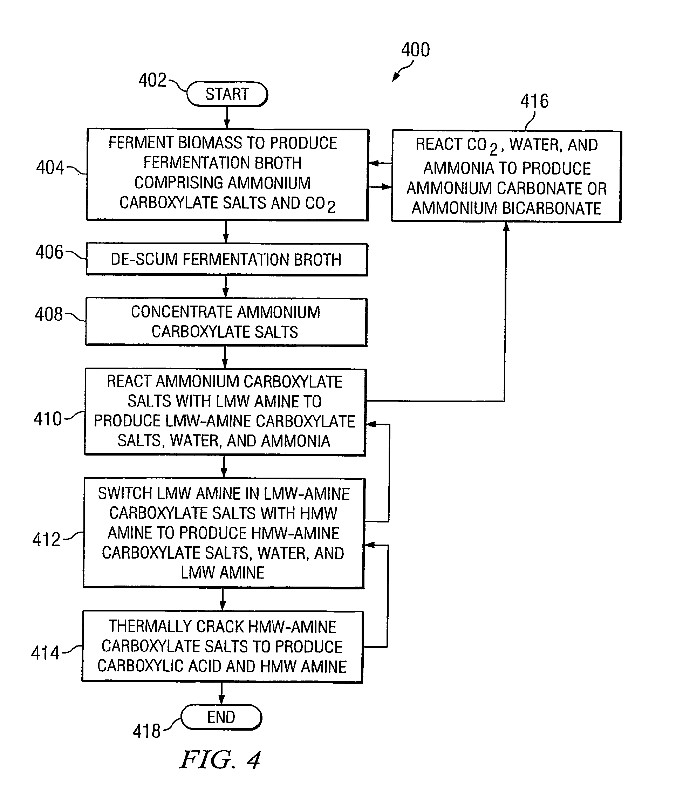

A better understanding of the process employed by fermentation system 300 may be had by making reference to FIGURE 4, which illustrates a flowchart 400 of a method of producing carboxylic acids from biomass utilizing the equipment shown in FIGURE 3. Flowchart 400 begins in step 402. At step 404, biomass is fermented to produce carbon dioxide and a fermentation broth comprising ammonium carboxylate salts. Generally, this is performed using a plurality of countercurrent fermentors utilizing an ammonium carbonate or ammonia bicarbonate buffer. The fermentation broth produced by the plurality of fermentors is then de-scummed at step 406. In particular embodiments, this may be performed using an ultrafilter that filters out the scum, or a coagulant or flocculant that causes the scum to form a precipitate that may then be filtered out.

At step 408, the de-scummed fermentation broth is then concentrated using a dewatering system, such as a vapor compression system. This dewatering system concentrates the fermentation broth into a nearly saturated (*i.e*., approximately 50%) solution of ammonium carboxylate salts. This nearly saturated solution of ammonium carboxylate salts is then reacted with LMW amine to produce LMW-amine carboxylate salts at step 410. As part of this process, water and ammonia are also given off. This water and ammonia may be reacted with carbon dioxide from the fermentors at step 416 to produce ammonium carbonate or ammonium bicarbonate that may be used to buffer the fermentation reaction inside the fermentors.

The LMW amine in the LMW-amine carboxylate salts from step 410 is then switched with HMW amine at step 412 to produce HMW-amine carboxylate salts and LMW amine. This LMW amine may then be used to produce more LMW-amine carboxylate salts at step 410. The HMW-amine carboxylate salts are then thermally cracked in a reactive distillation column to produce carboxylic acid and HMW amine. The HMW amine exits the bottom of the column and may be used to react with the LMW-amine carboxylate salts at step 412. The carboxylic acid exits the top of the distillation column and may be collected. At step 418, flowchart 400 terminates.

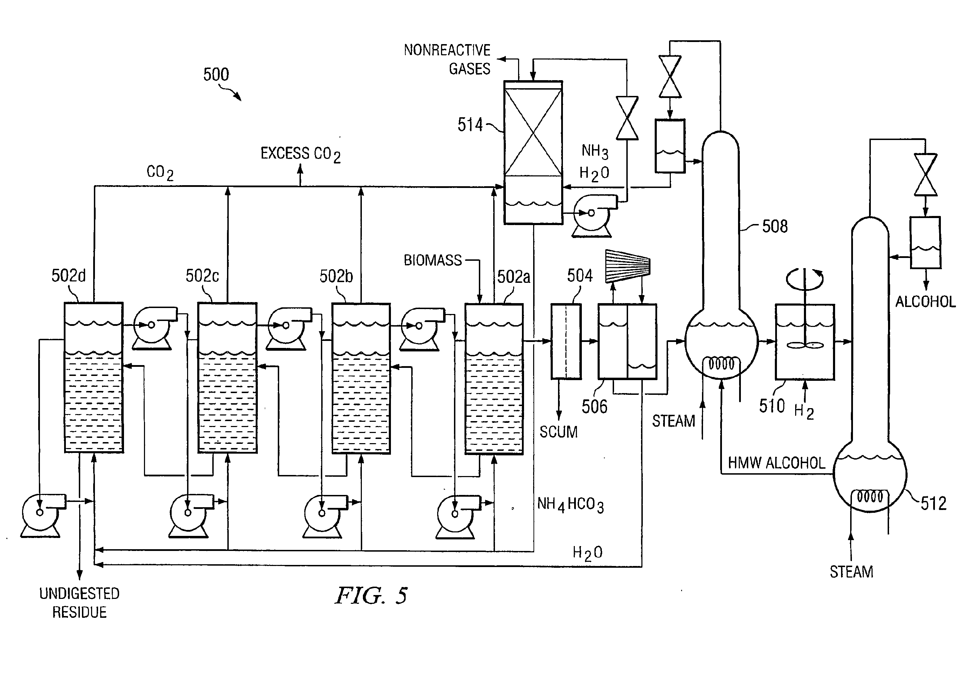

Unlike systems 100 (FIGURE 1) and 300 (FIGURE 3), which convert biomass into carboxylic acids, other embodiments may be utilized to convert biomass into alcohols. FIGURE 5 illustrates a fermentation system 500 in accordance with one such embodiment. As shown in FIGURE 5, fermentation system 500 comprises one or more fermentors 502, a dewatering system, distillation columns 508 and 512, a hydrogenation reactor 510, and a packed column 514.

Although any number of suitable fermentor geometries and arrangements may be used, FIGURE 5 illustrates fermentation system 500 comprising four countercurrent fermentors 502a-d in which fresh biomass is added to the top of fermentor 502a and fresh water is added to the bottom of fermentor 502d. Fermentation broth is ultimately harvested from fermentor 502a. These fermentors 502 operable similarly to fermentors 102 and 302 discussed above with regard to FIGURES 1 and 3, respectively.

Fermentation broth harvested from fermentor 502a is sent for downstream processing. In particular embodiments, this fermentation broth may also include scum that is undesirable in the downstream processing steps. In particular embodiments, this scum may be removed using any suitable method. For example, in particular embodiments, the fermentation broth may be pumped through an ultrafilter 504 having a molecular weight cut-off that allows ammonium carboxylic acid salts to pass but retains scum. In other embodiments, a coagulant or flocculant, such as those employed to clarify sugar juice extracted from sugarcane, may be added to the fermentation broth to cause a precipitate to form that may be removed by filtration.

The de-scummed fermentation broth from fermentors 502 is passed to a dewatering system 506, which removes water from the broth to form a nearly saturated solution (*i.e*., about 50%) of ammonium carboxylate salts. Although a variety of dewatering systems may be used, FIGURE 5 illustrates dewatering system 506 as a vapor-compression system that works similarly to vapor-compression systems discussed above with regard to FIGURES 1 and 3.

The concentrated ammonium carboxylate salts from dewatering system 506 are sent to a reactive distillation column 508 where they are mixed with a HMW alcohol having four or more carbons. In reactive distillation column 508, the ammonium carboxylate salts react with the alcohol to form a HMW ester, which stays at the bottom of the column. Typically, this reaction is operated under alkaline conditions. Reflux helps reduce the loss of HMW alcohol and HMW ester from the top of the column. Water and ammonia exiting the top of the column 508 are sent to a packed column 514, where they are reacted with carbon dioxide from fermentors 502 to form ammonium bicarbonate or ammonium carbonate, depending upon the pH maintained with the column, which may be used to buffer the solutions in fermentors 502. HMW esters exit the bottom of reactive distillation column 508 and are sent to a hydrogenation reactor 510 where they are converted into LMW and HMW alcohols. To promote the hydrogenation, particular embodiments of the present invention may employ a suitable catalyst, such as Raney nickel, platinum, or palladium. These alcohols are sent to distillation column 512, where they are separated. LMW alcohols exit the top of distillation column 512 where they may be collected, whereas HMW alcohols exit the bottom of column 512 and are recycled to reactive distillation column 508.

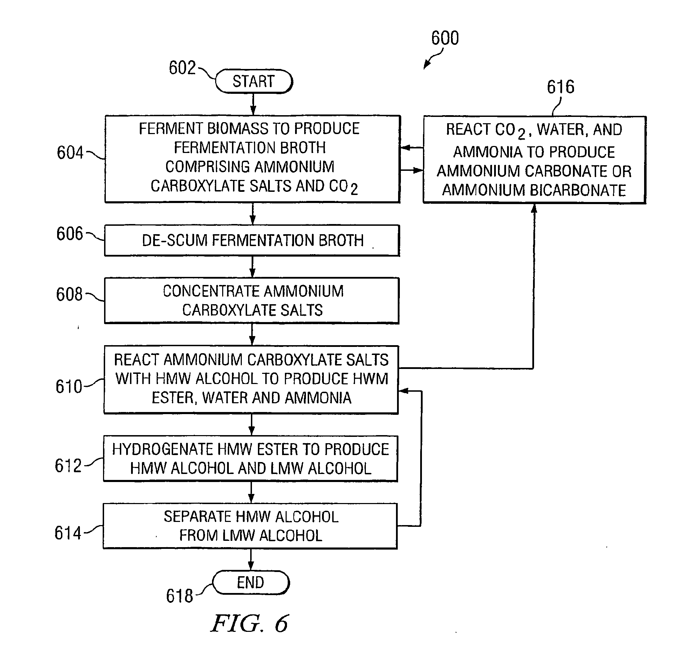

A better understanding of the process employed by fermentation system 500 may be had by making reference to FIGURE 6, which illustrates a flowchart 600 of a method of producing carboxylic acids from biomass utilizing the equipment shown in FIGURE 5. Flowchart 600 begins in step 602. At step 604, biomass is fermented to produce carbon dioxide and a fermentation broth comprising ammonium carboxylate salts. Generally, this is performed using a plurality of countercurrent fermentors utilizing an ammonium carbonate or ammonia bicarbonate buffer. The fermentation broth produced by the plurality of fermentors is then de-scummed at step 606. In particular embodiments, this may be performed using an ultrafilter that filters out the scum or a coagulant or flocculant that causes the scum to form a precipitate that may then be filtered out.

At step 608, the de-scummed fermentation broth is then concentrated using a dewatering system, such as a vapor-compression system. This dewatering system concentrates the fermentation broth into a nearly saturated (*i.e*., approximately 50%) solution of ammonium carboxylate salts. This nearly saturated solution of ammonium carboxylate salts is then reacted with HMW alcohols to produce HMW esters at step 610. As part of this process, water and ammonia are also given off. This water and ammonia may be reacted with carbon dioxide from the fermentors at step 616 to produce ammonium carbonate or ammonium bicarbonate that may be used to buffer the fermentation reactions inside the fermentors.

The HMW alcohols from step 610 are then hydrogenated at step 612 to produce both HMW alcohol and LMW alcohol. These alcohols are then separated in a distillation column at step 614. The HMW alcohol exits the bottom of the column and may be used to react with the ammonium carboxylate salts at step 610. The LMW alcohols exit the top of the column and maybe collected. At step 618, flowchart 600 terminates.

By buffering the fermentation reaction using ammonium carbonate or ammonium bicarbonate, the method of the present invention offre significant benefits over others methods. For example, if ammonia were added directly to the fermentors, the pH inside the fermentors could become too high and damage the microorganisms used to ferment the biomass. Additionally, the use of ammonium carbonate or ammonium bicarbonate buffers allow for simplified downstream processing of the fermentation broth, compared to calcium-based buffer systems where calcium salts may collect on the surfaces of heat exchangers and other equipment.

## Claims

1. A method of converting biomass comprising:
fermenting biomass in at least one fermentor to produce a fermentation broth comprising ammonium carboxylate salt;
reacting the ammonium carboxylate salt with a high-molecular-weight amine to produce amine carboxylate salt; and
thermally cracking the amine carboxylate salt to produce carboxylic acid;
wherein the fermentors contain a buffer selected from the group consisting of ammonium carbonate and ammonium bicarbonate.

2. The method of Claim 1, wherein the at least one fermentor comprises a plurality of countercurrent fermentors.

3. The method of Claim 1, further comprising producing the buffer by reacting carbon dioxide with water and ammonia released during the reaction of the ammonium carboxylate salt with the high-molecular-weight amine.

4. The method of Claim 1, further comprising concentrating the fermentation broth to concentrate the ammonium carboxylate salt prior to reacting the ammonium carboxylate salt with the high-molecular-weight amine.

5. The method of Claim 1, further comprising de-scumming the fermentation broth.

6. The method of Claim 1, wherein the fermentors contain a mixed culture of acid-forming microorganisms.

7. The method of Claim 6, wherein the microorganisms are at least one of:
(i) adapted to high-salt environment;
(ii) native to inoculum; and
(iii) native to soil or cattle rumen.

8. The method of Claim 1, wherein the fermentors are maintained at a pH between about 6.5 and about 7.5.

9. The method of Claim 1, wherein the fermentors contain a methane inhibitor.

10. The method of Claim 9, wherein the methane inhibitor is iodoform, bromoform, or bromoethane sulfonic acid.

11. The method of Claim 1, wherein the high-molecular-weight amine comprises tri-octyl amine or triethanol amine.

12. The method of Claim 1, wherein reacting the ammonium carboxylate salt includes
reacting the ammonium carboxylate salt with a low-molecular-weight amine to produce a low-molecular-weight-amine carboxylate salt;
switching the low-molecular-weight amine in the low-molecular-weight-amine carboxylate salt with a high-molecular-weight amine to form a high-molecular-weight-amine carboxylate salt.

13. The method of Claim 12, further comprising producing the buffer by reacting carbon dioxide with water and ammonia released during the reaction of the ammonium carboxylate salt with the low-molecular-weight amine.

14. The method of Claim 12; further comprising concentrating the fermentation broth to concentrate the ammonium carboxylate salt prior to reacting the ammonium carboxylate salt with the low-molecular-weight amine.

15. The method of Claim 12, wherein the low-molecular-weight amine is at least one of:
(i) a tertiary amine;
(ii) water soluble; and
(iii) triethyl amine, methyl diethyl amine, dimethyl ethanol amine, or ethanol amine.

16. The method of Claim 12, wherein the low-molecular-weight amine has a standard boiling point above about 100°C.

17. The method of Claim 1, wherein reacting the ammonium carboxylate salt includes:
reacting the ammonium carboxylate salt with a high-molecular-weight alcohol to produce a high-molecular-weight ester; and
hydrogenating the high-molecular weight ester to produce alcohol.

18. The method of Claim 17, further comprising separating the alcohol into low-molecular-weight alcohol and high-molecular-weight alcohol.

19. The method of Claim 18, wherein the high-molecular-weight alcohol comprises at least four carbons.

20. The method of Claim 17, further comprising producing the buffer by reacting carbon dioxide with water and ammonia released during the reaction of the ammonium carboxylate salt with the high-molecular-weight alcohol.

21. The method of Claim 17, further comprising concentrating the fermentation broth to concentrate the ammonium carboxylate salt prior to reacting the ammonium carboxylate salt with the high-molecular-weight alcohol.

22. The method of Claim 17, further comprising maintaining the fermentors at a pH between about 6.5 and about 7.5.

23. The method of Claim 17, wherein hydrogenating the high-molecular weight ester to produce alcohol comprises utilizing a catalyst.

24. The method of Claim 23, wherein the catalyst is Raney nickel, platinum, or palladium.

## Patentansprüche

1. Verfahren zur Umwandlung von Biomasse, umfassend:
das Fermentieren von Biomasse in mindestens einem Fermenter, um eine Fermentationsbrühe herzustellen, die Ammoniumcarboxylatsalz umfasst;
das Reagieren des Ammoniumcarboxylatsalzes mit einem hochmolekularen Amin, um Amincarboxylatsalz herzustellen; und
das Wärmecracken des Amincarboxylatsalzes, um Carbonsäure herzustellen;
wobei die Fermenter einen Puffer enthalten ausgewählt aus der Gruppe bestehend aus Ammoniumcarbonat und Ammonunbicarbonat.

2. Verfahren nach Anspruch 1, wobei der mindestens eine Fermenter mehrere Gegenstromfermenter umfasst.

3. Verfahren nach Anspruch 1, des Weiteren das Herstellen des Puffers durch Reagieren von Kohlendioxid mit Wasser und Ammoniak, das während der Reaktion des Ammoniumcarboxylatsalzes mit dem hochmolekularen Amin freigesetzt wird, umfassend.

4. Verfahren nach Anspruch 1, des Weiteren das Konzentrieren der Fermentationsbrühe umfassend, um das Ammoniumcarboxylatsalz vor dem Reagieren des Ammoniumcarboxylatsalzes mit dem hochmolekulare Amin zu konzentrieren.

5. Verfahren nach Anspruch 1, des Weiteren das Abschäumen der Fermentationsbrühe umfassend.

6. Verfahren nach Anspruch 1, wobei die Fermenter eine gemischte Kultur von säurebildenden Mikroorganismen enthalten.

7. Verfahren nach Anspruch 6, wobei die Mikroorganismen mindestens eines sind von:
(i) für eine hochsalzhaltige Umgebung geeignet;
(ii) im Inokulum natürlich vorkommend; und
(iii) im Boden oder in Rinderpansen natürlich vorkommend.

8. Verfahren nach Anspruch 1, wobei die Fermenter bei einem pH-Wert zwischen etwa 6,5 und etwa 7,5 gehalten werden.

9. Verfahren nach Anspruch 1, wobei die Fermenter einen Methaninhibitor enthalten.

10. Verfahren nach Anspruch 9, wobei der Methaninhibitor Iodoform, Bromoform oder Bromethansulfonsäure ist.

11. Verfahren nach Anspruch 1, wobei das hochmolekulare Amin Trioctylamin oder Triethanolamin umfasst.

12. Verfahren nach Anspruch 1, wobei das Reagieren des Ammoniumcarboxylatsalzes Folgendes umfasst
das Reagieren des Ammoniumcarboxylatsalzes mit einem niedermolekularen Amin, um ein niedermolekulares Amincarboxylatsalz herzustellen;
das Umtauschen des niedermolekularen Amins in dem niedermolekularen Amincarboxylatsalz durch ein hochmolekulares Amin, um ein hochmolekulares Amincarboxylatsalz zu bilden.

13. Verfahren nach Anspruch 12, des Weiteren das Herstellen des Puffers durch Reagieren von Kohlendioxid mit Wasser und Ammoniak, das während der Reaktion des Ammoniumcarboxylatsalzes mit dem niedermolekularen Amin freigesetzt wird, umfassend.

14. Verfahren nach Anspruch 12, des Weiteren das Konzentrieren der Fermentationsbrühe umfassend, um das Ammoniumcarboxylatsalz vor dem Reagieren des Ammoniumcarboxylatsalzes mit dem niedermolekularen Amin zu konzentrieren.

15. Verfahren nach Anspruch 12, wobei das niedermolekulare Amin mindestens eines ist von:
(i) einem tertiären Amin;
(ii) wasserlöslich; und
(iii) Triethylamin, Methyldiethylamin, Dimethylethanolamin oder Ethanolamin.

16. Verfahren nach Anspruch 12, wobei das niedermolekulare Amin einen Standardsiedepunkt von über etwa 100 °C aufweist.

17. Verfahren nach Anspruch 1, wobei das Reagieren des Ammoniumcarboxylatsalzes Folgendes umfasst:
das Reagieren des Ammoniumcarboxylatsalzes mit einem hochmolekularen Alkohol, um einen hochmolekularen Ester herzustellen; und
das Hydrieren des hochmolekularen Esters, um Alkohol herzustellen.

18. Verfahren nach Anspruch 17, des Weiteren das Trennen des Alkohols in niedermolekularen Alkohol und hochmolekularen Alkohol umfassend.

19. Verfahren nach Anspruch 18, wobei der hochmolekulare Alkohol mindestens vier Kohlenstoffatome umfasst.

20. Verfahren nach Anspruch 17, des Weiteren das Herstellen des Puffers durch Reagieren von Kohlendioxid mit Wasser und Ammoniak, das während der Reaktion des Ammoxaiumcarboxylatsalzcs mit dem hochmolekularen Alkohol freigesetzt wird, umfassend.

21. Verfahren nach Anspruch 17, des Weiteren das Konzentrieren der Fermentationsbrühe umfassend, um das Ammoniumearboxylatsalz vor dem Reagieren des Ammoniumcarboxylatsalzes mit dem hochmolekularen Alkohol zu konzentrierten.

22. Verfahren nach Anspruch 17, des Weiteren das Halten der Fermenter bei einem pH-Wert zwischen etwa 6,5 und etwa 7,5 umfassend.

23. Verfahren nach Anspruch 17, wobei das Hydrieren des hochmolekularen Esters zum Herstellen von Alkohol das Verwenden eines Katalysators umfasst.

24. Verfahren nach Anspruch 23, wobei der Katalysator Raney-Nickel, Platin oder Palladium ist.

## Revendications

1. Procédé de conversion de biomasse comprenant :
la fermentation de biomasse dans au moins un fermenteur pour produire un bouillon de fermentation comprenant un sel carboxylate d'ammonium ;
la réaction du sel carboxylate d'ammonium avec une amine de poids moléculaire élevé pour produire du sel carboxylate d'amine ; et
le craquage thermique du sel carboxylate d'amine pour produire de l'acide carboxylique ;
dans lequel les fermenteurs contiennent un tampon choisi par le groupe constitué du carbonate d'ammonium et du bicarbonate d'ammonium.

2. Procédé selon la revendication 1, dans lequel le au moins un fermenteur comprend une multitude de fermenteurs à contre-courant.

3. Procédé selon la revendication 1, comprenant en outre la production du tampon en faisant réagir du dioxyde de carbone avec de l'eau et l'ammoniac libéré durant la réaction du sel carboxylate d'ammonium avec l'amine de poids moléculaire élevé.

4. Procédé selon la revendication 1, comprenant en outre la concentration du bouillon de fermentation afin de concentrer le sel carboxylate d'ammonium avant la réaction du sel carboxylate d'ammonium avec l'amine de poids moléculaire élevé.

5. Procédé selon la revendication 1, comprenant en outre l'écumage du bouillon de fermentation.

6. Procédé selon la revendication 1, dans lequel les fermenteurs contiennent une culture mixte de micro-organismes formant de l'acide.

7. Procédé selon la revendication 6, dans lequel les micro-organismes sont au moins l'un parmi :
(i) ceux adaptés à l'environnement fortement salé ;
(ii) ceux d'origine naturelle dans l'inoculum ; et
(iii) ceux d'origine naturelle dans le sol ou dans le rumen de bovin.

8. Procédé selon la revendication 1, dans lequel les fermenteurs sont maintenus à un pH compris entre environ 6,5 et environ 7,5.

9. Procédé selon la revendication 1, dans lequel les fermenteurs contiennent un inhibiteur du méthane.

10. Procédé selon la revendication 9, dans lequel l'inhibiteur du méthane est iodoforme, bromoforme, ou l'acide bromoéthane sulfonique.

11. Procédé selon la revendication 1, dans lequel l'amine de poids moléculaire élevé est la trioctyl aminé ou la triéthanol amine.

12. Procédé selon la revendication 1, dans lequel la réaction du sel carboxylate d'ammonium inclut :
la réaction du sel carboxylate d'ammonium avec une amine de bas poids moléculaire pour produire un sel carboxylate aminé de faible poids moléculaire ;
le remplacement de l'amine de faible poids moléculaire dans le sel carboxylate aminé de faible poids moléculaire par une amine de poids moléculaire élevé afin de former un sel carboxylate aminé de poids moléculaire élevé.

13. Procédé selon la revendication 12, comprenant en outre la production du tampon en faisant réagir du dioxyde de carbone avec de l'eau et l'ammoniac libéré durant la réaction du sel carboxylate d'ammonium avec l'amine de faible poids moléculaire.

14. Procédé selon la revendication 12, comprenant en outre la concentration du bouillon de fermentation afin de concentrer le sel carboxylate d'ammonium avant la réaction du sel carboxylate d'ammonium avec l'amine de faible poids moléculaire.

15. Procédé selon la revendication 12, dans lequel l'amine de faible poids moléculaire est au moins l'une parmi :
(i) une amine tertiaire ;
(ii) une amine soluble dans l'eau ; et
(iii) la triéthylamine, méthyl diéthylamine, diméthyl éthanolamine ou l'éthanolamine.

16. Procédé selon la revendication 12, dans lequel l'amine de faible poids moléculaire a un point d'ébullition standard situé au-dessus d'environ 100 °C.

17. Procédé selon la revendication 1, dans lequel la réaction du sel carboxylate d'ammonium inclut :
la réaction du sel carboxylate d'ammonium avec un alcool de poids moléculaire élevé pour produire un ester de poids moléculaire élevé ;
l'hydrogénation de l'ester de poids moléculaire élevé pour produire de l'alcool.

18. Procédé selon la revendication 17, comprenant en outre la séparation de l'alcool en alcool de faible poids moléculaire et alcool de poids moléculaire élevé.

19. Procédé selon la revendication 18, dans lequel l'alcool de poids moléculaire élevé comprend au moins quatre atomes de carbone,

20. Procédé selon la revendication 17, comprenant en outre la production du tampon en faisant réagir du dioxyde de carbone avec de l'eau et l'ammoniac libéré durant la réaction du sel carboxylate d'ammonium avec l'alcool de poids moléculaire élevé.

21. Procédé selon la revendication 17, comprenant en outre la concentration du bouillon de fermentation afin de concentrer le sel carboxylate d'ammonium avant de faire réagir le sel carboxylate d'ammonium avec l'alcool de poids moléculaire élevé.

22. Procédé selon la revendication 17, comprenant en outre le maintien des fermenteurs à un pH compris entre environ 6,5 et environ 7,5.

23. Procédé selon la revendication 17, dans lequel l'hydrogénation de l'ester de poids moléculaire élevé pour produire l'alcool comprend l'utilisation d'un catalyseur.

24. Procédé selon la revendication 23, dans lequel le catalyseur est le nickel de Raney, le platine ou le palladium.
